# EUROPEAN PATENT APPLICATION

(11) **EP 4 085 906 A1**
(43) Date of publication of application: **09.11.2022**
(21) Application number: 21305586.6
(22) Date of filing: 06.05.2021
(51) Int. Cl.: A61K 31/195, A61K 31/216, A61K 31/37, A61K 45/06, A61P 9/00, A61P 43/00

(54) **COMPOSITION COMPRISING PPAR-MODULATOR AND AN UROLITHIN DERIVATIVE AND USES THEREOF**

(71) Applicant: UNIVERSITE DE BORDEAUX, 33000 Bordeaux (FR); Centre Hospitalier Universitaire de Bordeaux, 33404 Talence Cedex (FR); INSTITUT NATIONAL DE LA SANTE ET DE LA RECHERCHE MEDICALE - INSERM, 75013 Paris (FR)
(72) Inventor: ROSSIGNOL, Rodrigue, 33600 PESSAC (FR); DARD, Laetitia, 33700 MERIGNAC (FR); LACOMBE, Didier, 33270 FLOIRAC (FR); DIAS AMOEDO, Nivea, 33600 PESSAC (FR)
(74) Representative: Novagraaf Technologies

(57) **Abstract**

The present invention belongs to the field of medicine, and more precisely to the field of medecine useful in the treatment of cardiac diseases with contributing mitochondrial dysfunction.

The present invention relates to a composition a pharmaceutical composition comprising a PPAR-modulator and an urolithin derivative of formula I: 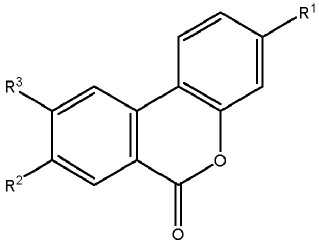 wherein R¹, R² and R³ independently represents H or OH and its use thereof in the treatment of various cardiac diseases linked to cardiac diseases with contributing mitochondrial dysfunction.

## Description

### Technical field

The present invention belongs to the field of medicine, and more precisely to the field of medicine useful in the treatment of cardiac diseases caused by energy metabolism dysfunction.

The present invention relates to a pharmaceutical composition comprising a PPAR-modulator and an urolithin derivative and its use thereof in the treatment of various cardiac diseases linked to cardiac diseases with contributing mitochondrial dysfunction, such as the Costello Syndrome for instance.

### Technical background

According to the American Center for Diseases Control and Prevention, heart disease is the leading cause of death for men, women, and people of most racial and ethnic groups in the United States. One person dies every 36 seconds in the United States from cardiovascular disease and about 655,000 Americans die from heart disease each year - that's 1 in every 4 deaths. Heart disease costs the United States about $219 billion each year from 2014 to 2015. This includes the cost of health care services, medicines, and lost productivity due to death.

Mitochondria have emerged as a central factor in the pathogenesis and progression of heart failure, and other cardiovascular diseases, as well, but no therapies are available to prevent, to reduce or to treat mitochondrial dysfunction. The National Heart, Lung, and Blood Institute convened a group of leading experts in heart failure, cardiovascular diseases, and mitochondria research in August 2018 **[1].** These experts concluded that mitochondrial dysfunction and energy deficiency are strongly implicated in the development of hypertrophic cardiomyopathy and of heart failure **[2].**

Despite the use of guideline-directed therapies, the morbidity and mortality of heart failure remains unacceptably high. Novel therapeutic approaches are thus greatly needed. In this context, targeting mitochondrial dysfunction in heart disease may provide novel approaches for the prevention and the treatment of cardiac diseases.

The causes of heart failure are numerous and include, for instance, coronary artery disease (atherosclerosis), past heart attack (myocardial infarction), high blood pressure (hypertension or HBP), abnormal heart valves, heart muscle disease (dilated cardiomyopathy, hypertrophic cardiomyopathy) or inflammation (myocarditis), severe lung disease, diabetes, obesity, sleep apnea and heart defects present at birth (congenital heart disease). A large number of rare genetic defects are responsible for cardiac disease and heart failure, among other symptoms. Such pathologies are for instance the mitochondrial diseases, or RASopathies.

Germline mutations that activate RAS/MAPK signaling are responsible for the 'RASopathies', a group of rare human developmental diseases that affect more than 400,000 individuals in the United States alone **[3].** Costello syndrome (CS) was the first RASopathy discovered in 1971 **[4]** and was described as a multiple congenital anomaly syndrome caused by heterozygous activating germline mutations in *HRAS.* Most individuals affected by CS carry a mutation in HRAS at the G12 position, and more than 80% of CS individuals have a p.G12S substitution. The second most-common substitution is p.G12A **[5-6].** These mutations maintain HRAS in a constitutively (over)active state **[7].** Because of the RAS/MAPK pathway activation, development is altered and most children with CS exhibit an increased birth weight, dysmorphic craniofacial features, failure to thrive and gastro-esophageal reflux with oral aversion, especially during the newborn period. CS can also involve the skin with excessive wrinkling and redundancy over the dorsum of the hands and feet, along with deep plantar and palmar creases **[8],** as well as an increased risk of developing benign or malignant tumors **[8-10]**.

A central feature of CS and other RASopathies is hypertrophic cardiomyopathy (HCM) **[11-12].** Musculoskeletal abnormalities such as hypotonia were also reported in CS. Furthermore, HCM was observed in transgenic mutant HRAS mouse models or in patients expressing constitutively active forms of HRAS **[13-18],** but the molecular mechanisms linking HRAS activation with cardiac or skeletal muscle dysfunction remain unknown **[19-20].** However, cardiac involvement remains a major determinant for the prognosis of CS **[21],** raising the need to propose adapted therapeutic strategies able to improve the quality of life of these patients and reduce complications. Several lines of evidence have indicated that HCM typically associates with energetic insufficiency caused by bioenergetic alterations of the heart **[22-23].** In particular, genetic defects in oxidative phosphorylation (OXPHOS) and fatty oxidation (FAO) machineries can lead to HCM **[24].** According to the American Heart Association, 'alterations in mitochondrial function are increasingly being recognized as a contributing factor in myocardial infarction and in patients presenting with cardiomyopathy' **[25].** Therefore, we hypothesized that CS could include early alterations of heart bioenergetics participating to the development of HCM. This hypothesis was also based on a prospective screening of 18 patients with various Ras-MAPK pathway defects that detected biochemical signs of disturbed OXPHOS **[26],** and on the partial overlap of clinical manifestations between mitochondrial diseases and RASopathies **[27].**

Heart bioenergetics strongly relies on OXPHOS, and the heart-beating function is linearly dependent on the flux of mitochondrial ATP synthesis **[28-29].** Moreover, the regulation of mitochondrial turnover, including organelle biogenesis and degradation, is a major site of bioenergetic control in heart physiology **[30-31].** Mitochondrial biogenesis requires the coordinated expression of a set of 1,136 genes controlled, to a large extent, by the master regulator 5'AMP-activated protein kinase (AMPK) **[32].** Briefly, AMPK stimulation induces activation of peroxisome proliferator-activated receptor gamma coactivator 1-alpha (PGC1α), a transcriptional co-activator that promotes mitochondrial biogenesis in cooperation with specific transcription factors **[33].** AMPK activation also promotes mitochondrial degradation in conjunction with organelle biogenesis, resulting in a net increase in mitochondrial quality and function **[34-35].** The central role of AMPK and mitochondrial turnover in HCM pathophysiology was demonstrated *in vivo* using transgenic mouse knockout for AMPKα2 **[36],** PGC1α **[30-31],** NRF2 **[37],** ERRα **[38]** or PPARα [39]. All mouse models developed HCM (at rest or under stress) caused by defective mitochondrial turnover and bioenergetics. Finally, mitochondrial biogenesis is a druggable process, and pharmacological activators of AMPK or PGC1α prevent HCM development in preclinical studies **[40-43],** Yet, there is no treatment available in the clinics to stimulate mitochondrial energy metabolism in the heart and to rescue a reduction in mitochondrial content and/or a defective organelle turnover and quality control. There is a major unmet need to prevent and rescue mitochondrial dysfunction for the prevention and treatment of cardiac disease in RASOpathies and mitochondrial diseases of genetic or chemical origin **[1].** There is therefore a need for a composition that solves the above listed problems.

### Detailed description of the invention

Applicant has developed new pharmaceutical composition, comprising a combination of compounds that may be used as treatment to stimulate mitochondrial turnover and energy metabolism in the heart, and other tissues as well, and to rescue a reduction in mitochondrial efficacy and/or a defective organelle bioenergetics, biogenesis, turnover, dynamics and quality control. The composition according to the invention therefore meets the need to prevent and rescue mitochondrial dysfunction for the prevention and treatment of cardiac disease not only in RASopathies and mitochondrial diseases, but also in cardiopathies with a contributing bioenergetic deficiency resulting from mitochondrial alterations in organelle composition, turnover, quality control, signalling, dynamics, fueling and efficacy and REDOX homeostasis. The composition according to the invention therefore solves partially or completely the problems listed above.

The composition according to the invention comprises PPAR-modulator and an urolithin derivative.

The composition according to the invention has proven useful as a medicine, and in particular in the treatment of cardiac diseases with contributing mitochondrial dysfunction.

Thus, an object of the invention is a pharmaceutical composition comprising a PPAR-modulator and an urolithin derivative of formula I: 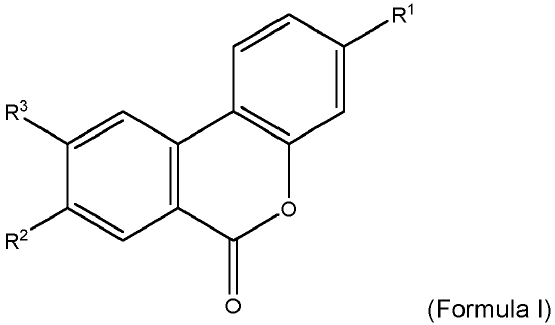 wherein R¹, R² and R³ independently represents H or OH.

Advantageously, the urolithin derivative of formula I may be urolithin A (R¹ and R² = OH and R³ = H), B (R¹ = OH and R² and R³ = H) and/or C (R¹, R² and R³ = OH): 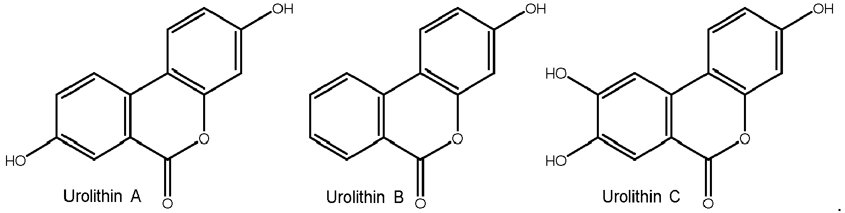 The urolithin derivative of formula I may preferably be Urolithin A.

In the context of the invention, it is meant by "PPAR-modulator", an agonist of any of or all of the different PPAR receptors.

Advantageously, the PPAR modulator may be chosen in the group comprising PPAR alpha agonists, PPAR gamma agonists, PPAR delta agonists, PPAR dual agonists (alpha/gamma or alpha/delta) and PPAR pan agonists (alpha/gamma/delta). The PPAR modulator may be selected from PPAR alpha agonists, PPAR gamma agonists, PPAR delta agonists, PPAR dual agonists (alpha/gamma or alpha/delta) and PPAR pan agonists (alpha/gamma/delta).

It is meant by "PPAR alpha agonists", a stimulator of PPAR alpha receptors-mediated signalling pathway.

It is meant by "PPAR gamma agonists", a stimulator of PPAR gamma receptors-mediated signalling pathway.

It is meant by "PPAR delta agonists", a stimulator of PPAR delta receptors-mediated signalling pathway.

Advantageously, the PPAR-modulator may be selected from any one or more from table 1 :

**Table 1: Example of PPAR-modulators according to the invention:**

| **PPAR alpha agonists** |
|---|
| Approved drugs - fibrates: fenofibrate, clofibrate, gemfibrozil, ciprofibrate, bezafibrate, Eupatilin (NSC 122413), Raspberry ketone |

| **PPAR gamma agonists** |
|---|
| drug class of thiazolidinediones; Actos (pioglitazone), Avandia (rosiglitazone), Pseudoginsenoside F11, Glabridin, MRL24, Leriglitazone |

| **PPAR delta agonists** |
|---|
| Cardarine (GW501516), L165041, GW0742 |

| **PPAR dual agonists** (alpha/gamma) |
|---|
| Muraglitazar, Tesaglitazar, Ragaglitazar, Naveglitazar, GW0742, |

| **PPAR pan agonists** (alpha/delta/gamma) |
|---|
| Lanifibranor (IVA-337), Bavachinin (7-O-Methylbavachin) |

| **PPAR dual agonists (alpha/delta)** |
|---|
| Elafibranor (GFT505) |

Advantageously, the PPAR-modulator may be any of the PPAR-modulator described in the review from Ichiro Takada & Makoto Makishima (2020) **[44],** the content of which is included by reference. Thus, the PPAR-modulator may be chosen in the group comprising fenofibrate ; bezafibrate ; L165041 ; GW0742 ; GW501516 ; pioglitazone ; rosiglitazone ; MRL24 ; 9-Hydroxy-10(E), 12(E)-octadecadienoic acid ; ethyl 4-hydroxy-3,3-methylpentanoate ; LY518674 ; hexadecanamide ; palmitoylethanolamide ; 9-octadecenamide ; GW7647 ; DY121 ; TZD salt ; 5-Hydroxy-4-phenylbutenolide ; benzoate ; phenylacetate ; MTTB ; INT131 ; LDT477 ; saroglitazar ; elafibranor ; lanifibranor ; and may be selected from any of the compounds the figure 3 from the above mentioned article of Ichiro Takada & Makoto Makishima (2020) **[44].**

Advantageously, the PPAR-modulator may preferably be selected from PPAR alpha agonists, and more preferably from fibrates. The PPAR-modulator may preferably be bezafibrate or fenofibrate, and more preferably bezafibrate.

Bezafibrate is also known as 2-(4-{2-[(4-chlorobenzoyl)amino]ethyl}phenoxy)-2-methylpropanoic acid (CAS 41859-67-0). Bezafibrate is marketed under the brand name Bezalip^{®} (and various other brand names). Bezafibrate is a fibrate drug that may be used as a lipid-lowering agent to treat hyperlipidaemia in adults and children. It is also known to help to lower LDL cholesterol and triglyceride in the blood and increase HDL.

Fenofibrate is also known as propan-2-yl 2-[4-(4-chlorobenzoyl)phenoxy]-2-methylpropanoate (49562-28-9). Fenofibrate, is marketed under various brand names such as Tricor, Fenoglide or Lipofen for example. Fenofibrate is a medication of the fibrate class used to treat abnormal blood lipid levels.

L165041 is also known as 4-[3-(4-Acetyl-3-hydroxy-2-propylphenoxy)propoxy]phenoxyacetic acid (CAS 79558-09-1).

GW0742 is also known as 4-[2-(3-Fluoro-4-trifluoromethyl-phenyl)-4-methyl-thiazol-5-ylmethylsulfanyl]-2-methyl-phenoxy}-acetic acid (CAS 317318-84-6).

GW501516 is also known as {4-[({4-methyl-2-[4-(trifluoromethyl)phenyl]-1,3-thiazol-5-yl}methyl)sulfanyl]-2-methylphenoxy}acetic acid (CAS 317318-70-0).

Pioglitazone is also known as 5-[[4-[2-(5-ethylpyridin-2-yl)ethoxy]phenyl]methyl]-1,3-thiazolidine-2,4-dione (CAS 111025-46-8).

Rosiglitazone is also known as 5-[[4-[2-[methyl(pyridin-2-yl)amino]ethoxy]phenyl]methyl]-1,3-thiazolidine-2,4-dione (CAS 155141-29-0).

MRL24 is also known as (S)-2-(3-((1-(4-Methoxybenzoyl)-2-methyl-5-(trifluoromethoxy)-1H-indol-3-yl)methyl)phenoxy)propanoic acid (CAS 393794-17-7).

9-Hydroxy-10(*E*), 12(*E*)-octadecadienoic acid is also known as (10*E*,12*Z*)-9-hydroxyoctadeca-10,12-dienoic acid (CAS 98524-19-7).

LY518674 is also known as 2-methyl-2-[4-[3-[1-[(4-methylphenyl)methyl]-5-oxo-4H-1,2,4-triazol-3-yl]propyl]phenoxy]propanoic acid (CAS 425671-29-0).

Hexadecanamide, its CAS number is 629-54-9.

Palmitoylethanolamide is also known as N-(2-Hydroxyethyl)hexadecanamide (CAS 544-31-0).

9-Octadecenamide, its CAS number is 3322-62-1.

GW7647 is also known as 2-[4-[2-[4-cyclohexylbutyl(cyclohexylcarbamoyl)amino]ethyl]phenyl]sulfanyl-2-methylpropanoic acid (CAS 265129-71-3).

TZD is also known as thiazolidinedione or 1,3-thiazolidine-2,4-dione (CAS 2295-31-0).

Concerning 5-Hydroxy-4-phenylbutenolide, "butenolide" is also know under its IUPAC name Furan-2(5*H*)-one.

MTTB is also known as (*E*)-2-(5-((4-methoxy-2-(trifluoromethyl)quinolin-6-yl)methoxy)-2-((4-(trifluoromethyl)benzyl)oxy)-benzylidene)-hexanoic acid.

Saroglitazar, its CAS number is 495399-09-2

Elafibranor is also known as GFT505.

Lanifibranor is also known as IVA-337.

Advantageously, the composition according to the invention may further comprise a pharmaceutically acceptable excipient. The pharmaceutically acceptable excipient may be chosen depending on the route of administration of the composition.

The composition according to the invention may be administrated by any common routes of administration. Routes of administration are generally classified by the location at which the substance/medicine/composition is applied. Advantageously, the composition according to the invention may be suitable for parenteral or enteral administration. Common examples include oral administration, intravenous injection, intramuscular injection, subcutaneous injection, topical administration. Preferably, the route of administration is oral administration or via a gastrointestinal probe (naso-gastric or gastrostomic procedures).

Advantageously, the composition according to the invention may be in the form of a solution (preferably an aqueous solution) or in the form of a powder (such as granulates or tablets).

Advantageously, when the composition according to the invention is a solution, the composition may comprise a solvent. The solvent may be for example water and/or an organic solvent. The solution may comprise a mixture of water and/or organic solvent(s). The solution may be reconstituted from the powder and solvent mixture comprising water and/or organic solvent(s).

Advantageously, the pharmaceutically acceptable excipient may be any excipient suitable for a pharmaceutical composition is the form of solution or powder. The pharmaceutically acceptable excipient may for example be chosen from the group comprising binders, disintegrants, fillers, dispersing agent, coating agents. Advantageously, the composition according to the invention may comprise from 12 to 1200 mg of PPAR-modulator, preferably PPAR alpha agonists, and more preferably fibrates. The PPAR-modulator may more preferably be bezafibrate or fenofibrate.

Advantageously, the composition according to the invention may comprise from 20 to 2000 mg of urolithin derivative of formula I, preferably urolithin A.

Advantageously, the composition according to the invention may comprise from 12 to 1200 mg of PPAR-modulator, preferably PPAR alpha agonists, and more preferably fibrates, and from 20 to 2000 mg of urolithin derivative of formula I, preferably urolithin A.

Advantageously, the composition according to the invention may comprise from 20 to 2000 mg of urolithin derivative of formula I, preferably urolithin A and from 12 to 1200 mg of PPAR-modulator, preferably PPAR alpha agonists, and more preferably fibrates. Preferably, the composition according to the invention may comprise from 20 to 2000 mg of Urolithin A and from 12 to 1200 mg of bezafibrate or fenofibrate, more preferably bezafibrate.

The composition of the invention may also be used as a medicine. The invention thus also encompasses the composition according to the invention for use as a medicine. The invention encompasses a therapeutic or prophylactic method of treatment comprising a step of administrating the composition according to the invention.

Advantageously, the composition according to the invention may be used in the treatment or the prevention of cardiac diseases with contributing mitochondrial dysfunction. Thus, the invention relates to a composition according to the invention for use in the treatment or the prevention of cardiac diseases with contributing mitochondrial dysfunction. The invention encompasses a therapeutic method of treatment or prevention of cardiac diseases with contributing mitochondrial dysfunction comprising a step of administrating the composition according to the invention.

It is meant by a "cardiac disease with contributing mitochondrial dysfunction" a heart disorder resulting in part from defective bioenergetics and altered mitochondrial REDOX and calcium signalling. The cardiac disease may be selected from cardiac diseases with contributing mitochondrial dysfunction of primary genetic origin, cardiac diseases with contributing mitochondrial dysfunction of secondary genetic origin and cardiovascular complication caused by mitotoxic iatrogenic effects of medicines.

Advantageously, the cardiac disease with contributing mitochondrial dysfunction of primary genetic origin may be selected from MELAS (Mitochondrial Encephalomyopathy Lactic Acidosis and Stroke-like Episodes), Leigh Syndrome MERRF (Myoclonic Epilepsy and Ragged-Red Fiber Disease), MIDD (Maternally inherited diabetes-deafness syndrome), NARP (Neuropathy, Ataxia, and Retinitis Pigmentosa), GRACILE (fetal growth restriction (GR), aminoaciduria (A), cholestasis (C), iron overload (I), lactacidosis (L), and early death (E)), MNGIE (Myoneurogastointestinal Disorder and Encephalopathy), Barth Syndrome, LHON (Leber Hereditary Optic Neuropathy), Pearson Syndrome, Kearns-Sayre Syndrome, CPEO (Chronic Progressive External Ophthalmoplegia Syndrome), Freiderich's Ataxia, CoQ10 deficiency, 3-Methylglutaconic acidurias, Sengers syndrome, Wolff-Parkinson White (WPW) syndrome, Congenital cataract-hypertrophic cardiomyopathy-mitochondrial myopathy syndrome, Beta-oxidation Defects (LCAD, LCHAD, MAD, MCAD, SCAD, SCHAD, VLCAD), Carnitine-Acyl-Carnitine Deficiency, Creatine Deficiency Syndromes, Leukodystrophy, alpers disease, Polymerase Gamma (POLG) Related Disorders. Preferably, the cardiac disease with contributing mitochondrial dysfunction of primary genetic origin may be selected from the Barth syndrome and the Beta-oxidation defects.

Advantageously, the composition for use in the treatment or prevention of a cardiac disease with contributing mitochondrial dysfunction of primary genetic origin, may be administrated at the dosage range/regimen of PPAR-modulator, preferably PPAR alpha agonists, more preferably fibrates and most preferably bezafibrate or fenofibrate, of from 12 to 1200 mg/day and urolithin derivative of formula I, preferably urolithin A of from 20 to 2000 mg/day.

Advantageously, the composition for use in the treatment or prevention of a cardiac disease with contributing mitochondrial dysfunction of primary genetic origin, may be administrated at the dosage range/regimen of bezafibrate or fenofibrate, preferably bezafibrate, of from of from 12 to 1200 mg/day and urolithin A of from 20 to 2000 mg/day.

Advantageously, the cardiac diseases with contributing mitochondrial dysfunction of secondary genetic origin may be selected from the Costello Syndrome, the cardio-faciocutaneous syndrome, the neurofibromatosis type 1, the Legius syndrome, the Noonan syndrome, the Noonan syndrome with multiple lentigines and the capillary malformation-arteriovenous malformation syndrome. The cardiac diseases caused by mitochondrial dysfunction of secondary genetic origin may preferably be the Costello syndrome.

Advantageously, the composition for use in the treatment or prevention of a cardiac disease with contributing mitochondrial dysfunction of secondary genetic origin, may be administrated at the dosage range/regimen of PPAR-modulator, preferably PPAR alpha agonists, more preferably fibrates and most preferably bezafibrate or fenofibrate of from of from 12 to 1200 mg/day and urolithin derivative of formula I, preferably urolithin A of from 20 to 2000 mg/day.

Advantageously, the composition for use in the treatment or prevention of a cardiac disease with contributing mitochondrial dysfunction of secondary genetic origin, may be administrated at the dosage range/regimen of bezafibrate or fenofibrate, preferably bezafibrate, of from of from 12 to 1200 mg/day and urolithin A of from 20 to 2000 mg/day.

Advantageously, the cardiovascular complication caused by mitotoxic iatrogenic effects of medicines may be caused by a medicine selected from alcoholism medications (such as disulfiram), analgesic and anti-inflammatory medications (such as aspirin, acetaminophen), diclofenac, fenoprofen, indomethacin, Naproxen, anesthetics (such as bupivacaine, lidocaine, propofol), angina medications (such as perhexiline), amiodarone, diethylaminoethoxyhexesterol (DEAEH), antiarrhythmic (such as amiodarone), antibiotics (such as tetracycline, antimycin A), antidepressants (such as amitriptyline, amoxapine, citalopram), fluoxetine, antipsychotics (such as chlorpromazine, fluphenazine, haloperidol, risperidone, quetiapine, clozapine, olanzapine), anxiety medications (such as alprazolam), diazepam, barbiturates (such as amobarbitalaprobarbital, butabarbital, butalbital hexobarbital, methylphenobarbital, pentobarbital, phenobarbital, primidone, propofol, secobarbital, thiobarbital), cholesterol medications, statins (such as atorvastatin, fluvastatin, lovastatin, pitavastatin, pravastatin, rosuvastatin, simvastatin), bileacids-cholestyramine, clofibrate, ciprofibrate, colestipol, colesevelam, cancer (chemotherapy) medications (such as mitomycinC, profiromycin, adriamycin (also called doxorubicin and hydroxydaunorubicin and included in the following chemotherapeuticregimens ABVD, CHOP, and FAC)), dementia medications (such as tacrine, galantamine) and diabetes medications (such as metformin, troglitazone, rosiglita-zone, buformin), HIV/AIDS medications (such as atripla). The cardiovascular complication may preferably be caused by mitotoxic iatrogenic effects of medicines selected from doxorubicin or statins.

Advantageously, the composition for use in the treatment or prevention of a cardiovascular complication caused by mitotoxic iatrogenic effects of medicines, may be administrated at the dosage range/regimen of PPAR-modulator, preferably PPAR alpha agonists, more preferably fibrates and most preferably bezafibrate or fenofibrate, of from of from 12 to 1200 mg/day and urolithin derivative of formula I, preferably urolithin A of from 20 to 2000 mg/day.

Advantageously, the composition for use in the treatment or prevention of a cardiovascular complication caused by mitotoxic iatrogenic effects of medicines, may be administrated at the dosage range/regimen of bezafibrate or fenofibrate, preferably bezafibrate, of from of from 12 to 1200 mg/day and urolithin A of from 20 to 2000 mg/day.

### Brief description of the figures

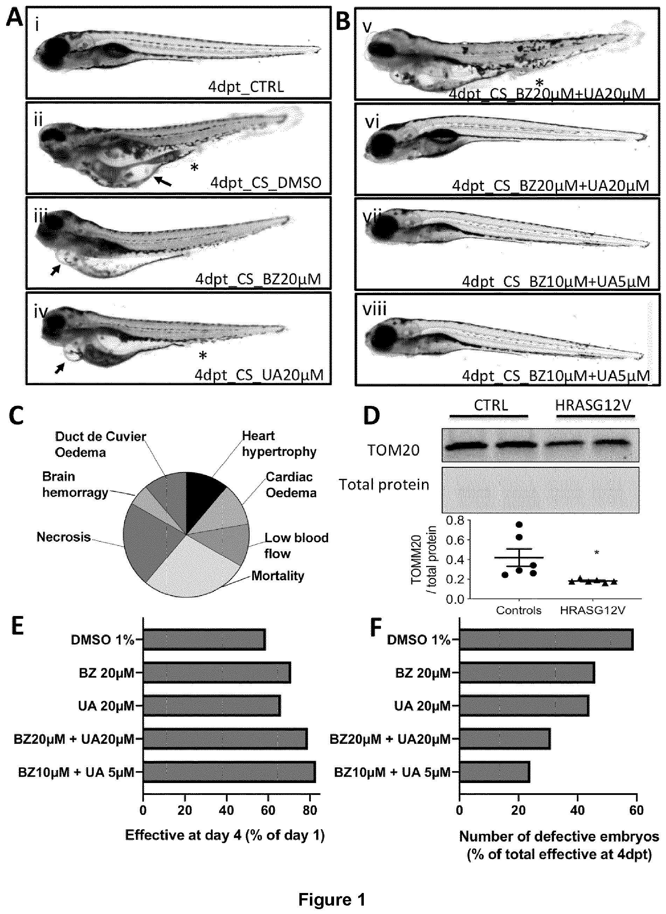

Figure 1 represents a combination of bezafibrate and urolithin A restore normal phenotype in Costello zebrafish. **(A-B).** Phenotype analysis of control (CTRL) zebrafish (i) or Costello (CS) zebrafish injected with HRASV12 plasmid (ii-viii) at 5 dpf, after 4 days of treatment (dpt) with DMSO (ii), bezafibrate (iii), urolithin A (iv) or bezafibrate (BZ) and urolithin A (UA) in combination (v-vii). Costello zebrafish developed cardiac edema (ii, iii, iv arrow), hemorrhages and vascularization defects (ii, iv, v star). Treatment with combination of 10µM BZ and 5µM UA significantly reduced the percentage of abnormal embryos (vii). **(C)** Description of the phenotypes observed in Costello zebrafish at 5dpf after expression of HRASV12 plasmid. **(D)** Expression level of the mitochondrial protein TOM20 in control or HRASG12V embryos. TOM20 protein content determined by westernblot was normalized to the total protein content. **(E)** Survival rate of Costello zebrafish after 4 days of treatment with DMSO, bezafibrate or urolithin A alone or in combination. Data were normalized to day 1 of treatment. **(F)** Percentage of defects appearance in Costello zebrafish after 4 days of treatment with DMSO, bezafibrate or urolithin A alone or in combination

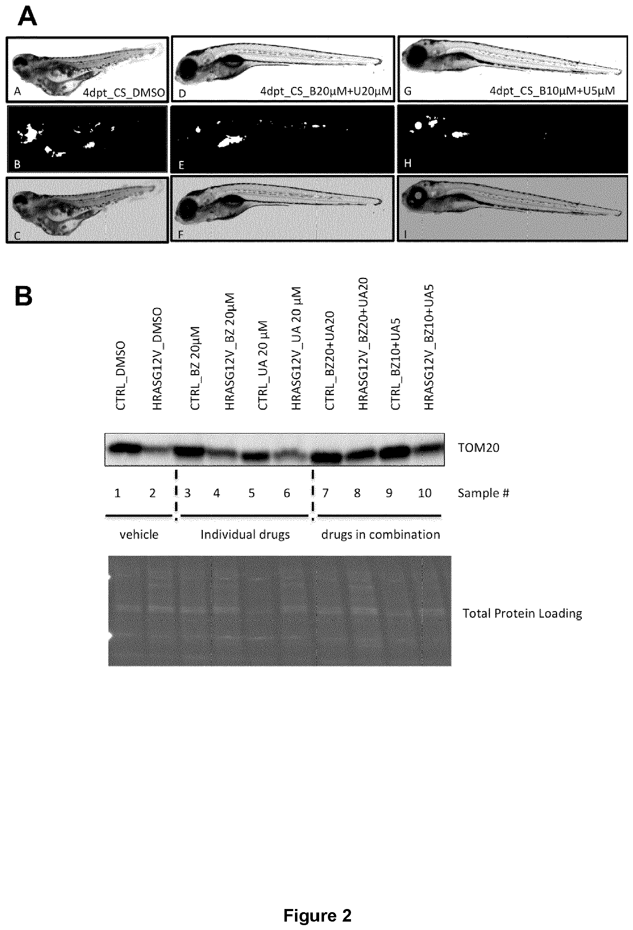

Figure 2 represents the CS zebrafish model molecular characterization. **A)** Observation of the expression of the HRASV12 plasmid in embryos at the 5 days post-fertilization stage and after 4 days of treatment. Embryos from Costello zebrafish animals showing cardiac edema (A-C). Embryos from Costello zebrafish animals showing no defect after a combined treatment with bezafibrate and urolithin A at 20µM (D-F) or 10µM and 5µM respectively (G-I) express the plasmid HRASV12: GFP at the same level as the embryos treated with DMSO. **B)** Measurement of TOM20 (mitochondrial protein marker of organelle content) was performed by westerblot on whole zebrafish. Injected control or Costello animals were used and denominated as CTRL or HRASG12V respectively. The zebrafish were treated with bezafibrate (BZ) or urolithin A (UA), alone or in combination. TOM20 expression was normalized to the total protein content in the different samples.

### EXAMPLES

### Generation, treatment and analysis of the Zebrafish model of the Costello syndrome.

Zebrafish (Danio rerio) embryos were obtained from natural spawnings and raised in embryo medium (E3) (97) at 28.5°C. Embryos were staged as described previously **[45].** Starting 5 days post-fertilisation (dpf), embryos were fed daily. All the studies conducted have been reviewed and approved (F341725 / 21063-2019061317218694). 1 nl of 12.5ng/µl of GFP-H-RASV12 plasmid and 12.5ng/µl of T2 transposase mRNA were co-injected in the first cell of zebrafish eggs. Control embryos were injected with the same volume of PBS. H-RASV12 was expressed in zebrafish embryo under heat shock, 30mn at 37°C at 1 day post fertilization (dpf) and embryo expressing GFP at 2 dpf were selected for the study.

The embryo injected with the HRASV12 plasmid were treated daily by bathing from 1dpf to 5dpf with respectively 20µM bezafibrate, 20µM urolithin A, 20µM bezafibrate + 20µM urolithin A, 10µM bezafibrate + 5µM urolithin A, 5µM bezafibrate + 5µM urolithin A. Control embryos were treated with the same volume of DMSO corresponding to 0.2% of DMSO. The imaging and phenotype analysis of mortality and defects of appearance after plasmid injection were performed on alive embryo anesthetized using ethyl 3-aminobenzoate methanesulfonate, MS-222 (Sigma, Cat.# A-5040) at 200 mg/L at 2 days post fertilization, 3 dpf, 4 dpf and 5 dpf using a ZEISS axiocam stereomicroscope (ZEISS, Germany).

### Example 1: Mitochondrial proteostasis and bioenergetic modulators restore mitochondrial homeostasis in Costello syndrome cell and animal models

Costello syndrome is a developmental disorder caused by germline gain-of-function mutations in the HRAS/MAPK pathway. The preclinical strategy of mitochondrial stimulation was thus tested at an early stage of the disease.

To this aim a *GFP-HRASV12* zebrafish model of CS **(****Figure 1****)** was generated, as previously described **[46].** Embryos injected with HRASV12 plasmid were selected at 2 days post fertilization (dpf) following their GFP^{+/+} expression. HRASV12 plasmid was activated under a heat shock of embryos at 37°C for 30 minutes. Embryos were observed daily from 2dpf to 5dpf in order to analyze the effect of HRASV12 overexpression on their physiological development **(****Figure 1A, B****).** At 2 dpf, 22% of the HRASG12V-embryos died and this rate increased to reach 60% mortality at 5dpf. At 5dpf, among alive embryos, 60% presented developmental defects similar to those observed in humans and mice **(****Figure 1C****)** as previously described **[46].** A cardiac phenotype characterized by heart hypertrophy (12%), cardia and pericardia edema (12%) associated with poorly developed heart and reduced blood flow (12%) was observed. Additional phenotypes were observed including brain hemorrhage (6%) and vascularization defect leading to edema in the duct of Cuvier or malformation of the aorta or the vein in the tail region around the urogenital opening (12%) **(****Figure 1Aii****).**

In order to analyse the impact of mitochondrial proteostasis modulators on the potential rescue of the observed HRASG12V-embryos developmental phenotype, bezafibrate and/or urolithin A were added to the zebrafish medium. Animal phenotyping revealed that the bezafibrate (BZ) or the urolithin A (UA) treatments increased the survival rate up to 70% and 65%, respectively, and decreased the number of defective individuals among alive embryo (45%, 43%) as compared to the vehicle-treated (DMSO) HRASG12V-embryos (60% of defective individuals). Interestingly, the combination of 10µM BZ and 5µM UA significantly increased the efficiency of the treatment by improving embryo survival up to +30% **(****Figure 1E****)** and reduced to a large extent (3-fold) the number of animals presenting genetic developmental defects **(****Figure 1F****),** as compared to vehicle-treated embryos. The expression of HRASV12 plasmid was verified by measuring the level GFP fluorescence emission which confirmed the stable expression of the transgene during the experiments and the absence of effect of the different treatments on plasmid expression **(****Figure 2A****).** From 1day post treatment (dpt) to 4 dpt, the GFP intensity remained constant in control embryos as well as in treated embryos. Last, the molecular investigation of the CS zebrafish model using western blot performed on whole embryos revealed a two-fold reduced expression of the mitochondrial marker TOM20 **(****Figure 1D****).**

The combination treatment composed of a composition comprising 10µM bezafibrate + 5µM urolithin A restored the expression level of TOM20 in the whole embryo and corrected the defective phenotype **(****Figure 2B****).**

These preclinical findings demonstrate the efficacy of combining urolithin A and bezafibrate to restore mitochondrial proteostasis and reduce the genetic developmental defects observed in a Zebrafish model of the Costello syndrome. These original findings suggest that mitochondrial proteostasis stimulation using a combination of urolithin A and bezafibrate could be used for the treatment of rare and common diseases with mitochondrial dysfunction.

### Example 2: CS zebrafish model molecular characterization

The expression of HRASV12 plasmid was verified by measuring the level GFP fluorescence emission which confirmed the stable expression of the transgene during the experiments and the absence of effect of the different treatments on plasmid expression **(****Figure 2A****).** The combination treatment composed of 10µM bezafibrate + 5µM urolithin A restored the expression level of TOM20 in the whole embryo and corrected the defective phenotype **(****Figure 2B****).**

These original findings suggest that mitochondrial proteostasis stimulation using a combination of urolithin A and bezafibrate could be used for the treatment of rare and common diseases with alteration of mitochondrial turnover.

### List of references

**[1]** Circulation. 2019;140:1205-1216
**[2]** doi: 10.1016/j.bbamcr.2010.09.006 / doi: 10.1056/NEJMra063052 / doi: 10.1172/JCI12084.
**[3]** Simanshu DK, Nissley D V., McCormick F. RAS Proteins and Their Regulators in Human Disease. Cell 2017;170(1):17-33.
**[4]** Costello J. A new syndrome. N Z Med J 1971;74:397.
**[5]** Aoki Y et al. Germline mutations in HRAS proto-oncogene cause Costello syndrome. [Internet]. Nat. Genet. 2005;37(10):1038-40.
**[6]** Tidyman WE, Rauen KA. Noonan, Costello and cardio-faciocutaneous syndromes: dysregulation of the Ras-MAPK pathway. Expert Rev. Mol. Med. 2008;10.
**[7]** Gibbs JB, Sigal IS, Poe M, Scolnick EM. Intrinsic GTPase activity distinguishes normal and oncogenic ras p21 molecules.. Proc. Natl. Acad. Sci. U. S. A. 1984;81(18):5704-5708.
**[8]** Siegel DH, Mann JA, Krol AL, Rauen KA. Dermatological phenotype in Costello syndrome: consequences of Ras dysregulation in development: Dermatological phenotype and Ras dysregulation in Costello syndrome. Br. J. Dermatol. 2012;166(3):601-607.
**[9]** Gripp KW. Tumor predisposition in Costello syndrome. Am. J. Med. Genet. Part C Semin. Med. Genet. 2005;137C(1):72-77.
**[10]** Estep AL, Tidyman WE, Teitell MA, Cotter PD, Rauen KA. HRAS mutations in Costello syndrome: detection of constitutional activating mutations in codon 12 and 13 and loss of wild-type allele in malignancy. Am. J. Med. Genet. A 2006;140(1):8-16.
**[11]** Lin AE et al. Clinical, pathological, and molecular analyses of cardiovascular abnormalities in Costello syndrome: A Ras/MAPK pathway syndrome. Am. J. Med. Genet. Part A 2011;155(3):486-507.
**[12]** Siwik ES, Zahka KG, Wiesner GL, Limwongse C. Cardiac disease in Costello syndrome. [Internet]. Pediatrics 1998;101(4 Pt 1):706-9.
**[13]** Wei B-R et al. Capacity for resolution of Ras-MAPK-initiated early pathogenic myocardial hypertrophy modeled in mice. [Internet]. Comp. Med. 2011;61(2):109-18.
**[14]** Hunter JJ, Tanaka N, Rockman HA, Ross J, Chien KR. Ventricular expression of a MLC-2v-ras fusion gene induces cardiac hypertrophy and selective diastolic dysfunction in transgenic mice. [Internet]. J. Biol. Chem. 1995;270(39):23173-8.
**[15]** Schuhmacher AJ et al. A mouse model for Costello syndrome reveals an Ang II-mediated hypertensive condition. [Internet]. J. Clin. Invest. 2008;118(6):2169-79.
**[16]** Gottshall KR et al. Ras-dependent pathways induce obstructive hypertrophy in echo-selected transgenic mice. [Internet]. Proc. Natl. Acad. Sci. U. S. A. 1997;94(9):4710-5.
**[17]** Sana ME et al. A Novel HRAS Mutation Independently Contributes to Left Ventricular Hypertrophy in a Family with a Known MYH7 Mutation. [Internet]. PLoS One 2016;11(12):e0168501.
**[18]** Oba D et al. Mice with an Oncogenic HRAS Mutation are Resistant to High-Fat Diet-Induced Obesity and Exhibit Impaired Hepatic Energy Homeostasis. [Internet]. EBioMedicine 2018;27:138-150.
**[19]** Rauen K. HRAS and the Costello syndrome. Clin. Genet. 2007;71(2):101-108.
**[20]** Gripp KW, Lin AE. Costello syndrome: a Ras/mitogen activated protein kinase pathway syndrome (rasopathy) resulting from HRAS germline mutations. Genet. Med. 2012;14(3):285-292.
**[21]** Hakim K, Boussaada R, Hamdi I, Msaad H. Cardiac events in Costello syndrome: One case and a review of the literature. [Internet]. J. Saudi Hear. Assoc. 2014;26(2): 105-9.
**[22]** Ormerod JOM, Frenneaux MP, Sherrid M V. Myocardial energy depletion and dynamic systolic dysfunction in hypertrophic cardiomyopathy [Internet]. Nat. Rev. Cardiol. 2016;13(11):677-687.
**[23]** Kloner RA et al. New and revisited approaches to preserving the reperfused myocardium. [Internet]. Nat. Rev. Cardiol. 2017;14(11):679-693.
**[24]** McManus MJ et al. Mitochondrial DNA Variation Dictates Expressivity and Progression of Nuclear DNA Mutations Causing Cardiomyopathy. [Internet]. Cell Metab. [published online ahead of print: August 23, 2018]; doi:10.1016/j.cmet.2018.08.002.
**[25]** Murphy E et al. Mitochondrial Function, Biology, and Role in Disease [Internet]. Circ. Res. 2016;118(12):1960-1991.
**[26]** Kleefstra T et al. Mitochondrial dysfunction and organic aciduria in five patients carrying mutations in the Ras-MAPK pathway. [Internet]. Eur. J. Hum. Genet. 2011;19(2):138-44.
**[27]** Dard L, Bellance N, Lacombe D, Rossignol R. RAS signalling in energy metabolism and rare human diseases. Biochim. Biophys. Acta - Bioenerg. [published online ahead of print: 2018]; doi:10.1016/j.bbabio.2018.05.003.
**[28]** Starling EH, Visscher MB. The regulation of the energy output of the heart. J. Physiol. 1927;62(3):243-261.
**[29]** Prebble JN. The discovery of oxidative phosphorylation: a conceptual off-shoot from the study of glycolysis. Stud. Hist. Philos. Sci. Part C Stud. Hist. Philos. Biol. Biomed. Sci. 2010;41(3):253-262.
**[30]** Arany Z et al. Transcriptional coactivator PGC-1α controls the energy state and contractile function of cardiac muscle [Internet]. Cell Metab. 2005;1(4):259-271.
**[31]** Kärkkäinen O et al. Heart specific PGC-1α deletion identifies metabolome of cardiac restricted metabolic heart failure [Internet]. Cardiovasc. Res. [published online ahead of print: June 20, 2018]; doi:10.1093/cvr/cvy155.
**[32]** (Nucleic Acids Research, Volume 49, Issue D1, 8 January 2021, Pages D1541-D1547, https://doi.org/10.1093/nar/gkaa1011).
**[33]** Rowe GC, Jiang A, Arany Z. PGC-1 coactivators in cardiac development and disease. [Internet]. Circ. Res. 2010;107(7):825-38.
**[34]** Fan J et al. Autophagy as a Potential Target for Sarcopenia. [Internet]. J. Cell. Physiol. 2016;231(7):1450-9.
**[35]** Bujak AL et al. AMPK Activation of Muscle Autophagy Prevents Fasting-Induced Hypoglycemia and Myopathy during Aging [Internet]. Cell Metab. 2015;21(6):883-890.
**[36]** Hu X et al. AMP Activated Protein Kinase- 2 Regulates Expression of Estrogen-Related Receptor- , a Metabolic Transcription Factor Related to Heart Failure Development [Internet]. Hypertension 2011;58(4):696-703.
**[37]** Erkens R et al. Left ventricular diastolic dysfunction in Nrf2 knock out mice is associated with cardiac hypertrophy, decreased expression of SERCA2a, and preserved endothelial function [Internet]. Free Radic. Biol. Med. 2015;89:906-917.
**[38]** Huss JM et al. The nuclear receptor ERRalpha is required for the bioenergetic and functional adaptation to cardiac pressure overload. [Internet]. Cell Metab. 2007;6(1):25-37.
**[39]** Luptak I et al. Decreased contractile and metabolic reserve in peroxisome proliferator-activated receptor-alpha-null hearts can be rescued by increasing glucose transport and utilization. [Internet]. Circulation 2005;112(15):2339-46.
**[40]** Fan Y et al. Resveratrol Ameliorates Cardiac Hypertrophy by Down-regulation of miR-155 Through Activation of Breast Cancer Type 1 Susceptibility Protein. [Internet]. J. Am. Heart Assoc. 2016;5(4). doi:10.1161/JAHA.115.002648.
**[41]** Meng R-S et al. Adenosine monophosphate-activated protein kinase inhibits cardiac hypertrophy through reactivating peroxisome proliferator-activated receptor-a signaling pathway [Internet]. Eur. J. Pharmacol. 2009;620(1-3):63-70.
**[42]** Thandapilly SJ et al. Resveratrol Prevents the Development of Pathological Cardiac Hypertrophy and Contractile Dysfunction in the SHR Without Lowering Blood Pressure [Internet]. Am. J. Hypertens. 2010;23(2):192-196.
**[43]** Huang Y et al. The PPAR pan-agonist bezafibrate ameliorates cardiomyopathy in a mouse model of Barth syndrome [Internet]. Orphanet J. Rare Dis. 2017;12(1):49.
**[44]** Takada & Makoto Makishima (2020), a patent review (2014-present), Expert Opinion on Therapeutic Patents, 30:1, 1-13, DOI: 10.1080/13543776.2020.1703952;
**[45]** Kimmel CB, Ballard WW, Kimmel SR, Ullmann B, Schilling TF. Stages of embryonic development of the zebrafish. [Internet]. Dev. Dyn. 1995;203(3):253-310.
**[46]** Santoriello C et al. Expression of H-RASV12 in a zebrafish model of Costello syndrome causes cellular senescence in adult proliferating cells [Internet]. Dis. Model. Mech. 2009;2(1-2):56-67.

## Claims

1. A pharmaceutical composition comprising a PPAR-modulator and an urolithin derivative of formula I: 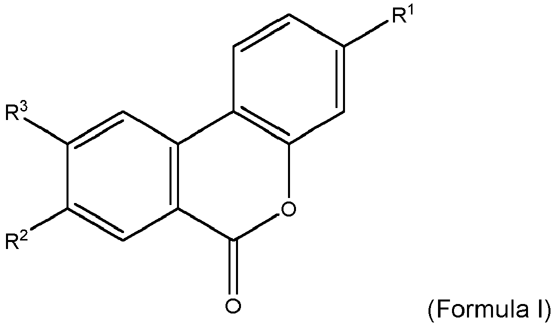 wherein R¹, R² and R³ independently represents H or OH.

2. The composition according to claim 1, wherein the urolithin derivative of formula I is urolithin A, B and/or C, preferably urolithin A.

3. The composition according to claim 1 or 2, wherein the PPAR-modulator is chosen in the group comprising PPAR alpha agonists, PPAR gamma agonists, PPAR delta agonists, PPAR dual agonists (alpha/gamma or alpha/delta) and PPAR pan agonists (alpha/gamma/delta).

4. The composition according to any of claims 1 to 3, wherein the PPAR-modulator is bezafibrate or fenofibrate, preferably bezafibrate.

5. The composition according to any of preceding claims, further comprising a pharmaceutically acceptable excipient.

6. The composition according to any of preceding claims, comprising from 12 to 1200 mg of PPAR-modulator.

7. The composition according to any of preceding claims, comprising from 20 to 2000 mg of urolithin derivative.

8. The composition according to any of preceding claims for use as a medicine.

9. The composition according to any of claims 1 to 7 for use in the treatment or prevention of cardiac diseases with contributing mitochondrial dysfunction.

10. The composition for use according to preceding claim, wherein the cardiac disease is selected from cardiac diseases with contributing mitochondrial dysfunction of primary genetic origin, cardiac diseases with contributing mitochondrial dysfunction of secondary genetic origin and cardiovascular complication caused by mitotoxic iatrogenic effects of medicines.

11. The composition for use according to preceding claim, wherein the cardiac diseases with contributing mitochondrial dysfunction of secondary genetic origin is the Costello syndrome.

12. The composition for use according to claim 10, wherein the cardiac disease is a cardiac disease with contributing mitochondrial dysfunction of primary genetic origin and wherein the composition is administrable at a dosage range/regimen of PPAR-modulator, preferably PPAR alpha agonists, more preferably fibrates and most preferably bezafibrate or fenofibrate, of from of from 12 to 1200 mg/day and urolithin derivative of formula I, preferably urolithin A of from 20 to 2000 mg/day.

13. The composition for use according to claim 10 or 11, wherein the cardiac disease is a cardiac disease with contributing mitochondrial dysfunction of secondary genetic origin and wherein the composition is administrable at a dosage range/regimen of PPAR-modulator, preferably PPAR alpha agonists, more preferably fibrates and most preferably bezafibrate or fenofibrate, of from of from 12 to 1200 mg/day and urolithin derivative of formula I, preferably urolithin A of from 20 to 2000 mg/day.

14. The composition for use according to claim 10, wherein the cardiac disease is a cardiac disease caused is a cardiovascular complication caused by mitotoxic iatrogenic effects of medicines and wherein the composition is administrable at a dosage range/regimen of PPAR-modulator, preferably PPAR alpha agonists, more preferably fibrates and most preferably bezafibrate or fenofibrate, of from of from 12 to 1200 mg/day and urolithin derivative of formula I, preferably urolithin A of from 20 to 2000 mg/day.
